# EUROPEAN PATENT APPLICATION

(11) **EP 4 763 254 A1**
(43) Date of publication of application: **24.06.2026**
(21) Application number: 24222400.4
(22) Date of filing: 20.12.2024
(51) Int. Cl.: A61M 15/00, A61M 15/06

(54) **INHALER**

(71) Applicant: air up group GmbH, 81671 München (DE)
(72) Inventor: JÄGER, Tim, 81245 München (DE)
(74) Representative: Bardehle Pagenberg Partnerschaft mbB Patentanwälte Rechtsanwälte

(57) **Abstract**

The present disclosure relates to an inhaler (1) for providing a substance (62) to a user for inhaling, in particular a medication substance such as a substance for the treatment of respiratory diseases, in particular chronic respiratory diseases, the inhaler comprising: a mouthpiece (11), an aroma channel (16) for being connected with an aroma source (50); and a reservoir (60) for accommodating at least the substance (62); wherein the inhaler is configured such that, when a user sucks on the mouthpiece: a main fluid (2) based on a gas such as air mixed with the substance can be provided to the mouth of the user for inhaling the substance through the mouthpiece; and an aroma fluid (3) based on a gas such as air and comprising an aroma component (52) generated from the aroma source (50), can be provided to the mouth of the user via the aroma channel (16).

## Description

### Technical field

The present invention relates to an inhaler for delivering a substance, particularly a medication, to a user for inhalation. The inhaler can be used for the treatment of respiratory diseases, including chronic respiratory diseases.

### Technical background

Respiratory diseases, in particular chronic respiratory diseases such as asthma and Chronic Obstructive Pulmonary Disease (COPD) affect millions of people worldwide, significantly impacting their quality of life. These conditions require ongoing management and therapy, often involving the use of inhalers to deliver medication directly to the lungs. Traditional inhalers, while effective in delivering medication, present several challenges that can hinder patient adherence and overall treatment efficacy.

Traditional inhalers often have an unpleasant taste, which can discourage patients from using them consistently. This can lead to poor adherence to the prescribed therapy, reducing its effectiveness and potentially worsening the patient's condition. Inhalers and other medical devices are often bulky and conspicuous, making patients feel self-conscious when using them in public. The stigma associated with using these devices can lead to patients avoiding their use in social settings, which can compromise their health. Proper use of inhalers often requires precise coordination between inhalation and device activation, which can be challenging for many patients, especially children and the elderly. Incorrect usage can result in inadequate delivery of medication, reducing its efficacy and potentially leading to exacerbations of the disease.

Moreover, many existing inhalers and nebulizers are large and not easily portable, making it difficult for patients to carry and use them throughout the day. This inconvenience can lead to inconsistent use, particularly when patients are away from home. Traditional treatments do not offer much in the way of customization to fit individual patient needs and preferences. A lack of personalization can result in suboptimal treatment outcomes and reduced patient satisfaction.

There is thus a need for improvement. Against this background, an object of the present invention is to address one or more or all of the above-mentioned disadvantages. Particularly, it is an object of the present invention to provide an improved inhaler. The improved inhaler should provide a comprehensive solution for the treatment diseases, in particular chronic respiratory diseases such as asthma and COPD.

### Summary of the invention

The above-mentioned objects and other objects, which become apparent from the following description, are solved by the subject-matter of the independent claims. Preferred embodiments are subject of the dependent claims.

### Inhaler

A **1^{st} embodiment** of the invention is directed to an inhaler for providing a substance to a user for inhaling, in particular a medication substance such as a substance for the treatment of respiratory diseases, in particular chronic respiratory diseases, the inhaler comprising: a mouthpiece, an aroma channel for being connected with an aroma source; and a reservoir for accommodating at least the substance; wherein the inhaler is configured such that, when a user sucks on the mouthpiece: a main fluid based on a gas such as air mixed with the substance can be provided to the mouth of the user for inhaling the substance through the mouthpiece; and an aroma fluid based on a gas such as air and comprising an aroma component generated from the aroma source, can be provided to the mouth of the user via the aroma channel.

In this manner, an improved inhaler for providing a substance to a user for inhaling is provided. This configuration may advantageously allow the user to simultaneously inhale medication and enjoy an aroma, potentially enhancing the user experience and compliance with the treatment regimen.

The inhaler (or more generally the device) may be designed to deliver a substance, particularly a medication substance for treating respiratory conditions, to a user through inhalation.

When the user inhales through the mouthpiece, the inhaler may be capable to deliver two types of fluids. The first may be a main fluid, which may be a gas such as air mixed with the medication substance, allowing the user to inhale the medication. The second may be an aroma fluid, which may also be a gas like air, but which may contain an aroma component derived from the aroma source. This aroma fluid may be delivered to the user through the aroma channel.

The mouthpiece may have the function that the user can communicate therewith using, e.g., their mouth in order to inhale the substance. The mouthpiece may be for instance a component of the inhaler or a separate component.

The aroma channel may be useful for guiding and/or diffusing aromatic compounds to enhance sensory experience. It may form part of the inhaler or of a different component, which may be connected with the inhaler. The aroma channel may thus contribute to a scent to be provided to the user, enhancing the experience. The aroma channel may be implemented in various ways, including but not limited to a groove, a slit, a cut, a vent and/or a more complex path. The specific implementation may help to evenly distribute the aroma within a space or the like. Generally, the aroma channel may facilitate that said aroma fluid can be provided to the user.

The aroma source may comprise an aroma container as described elsewhere herein. The aroma source may be mountable on the inhaler or a different component. The aroma source may be in fluid communication with the aroma channel.

The reservoir may provide for a storage for accommodating at least the substance but is not limited thereto. The reservoir may allow for accommodating more than just the substance. It is encompassed that the reservoir may accommodate, e.g., a pre-mixed main fluid, e.g., a gas such as air mixed with the substance. As the reservoir has the function to accommodate at least the substance, it is not necessary that the reservoir stores at least the substance for a prolonged time. It may be encompassed that the reservoir acts as a channel or the like for providing at least the substance or a premixed main fluid. The reservoir may be within the inhaler but is not limited thereto. Particularly, the reservoir may accommodate the medication substance to be inhaled by the user.

The main fluid may be a mixture of air and the substance that is inhaled by the user. The aroma fluid may be a mixture of air and the aroma component that is inhaled by the user through the aroma channel.

The advantages of this configuration include the ability to deliver both the medication substance and an aroma component, essentially simultaneously, which can enhance the user's experience and potentially improve compliance with the treatment regimen. The inclusion of an aroma component may make the inhalation process more pleasant and could help mask any unpleasant tastes or odors associated with the medication substance. This dual delivery system can be particularly beneficial for users with chronic respiratory diseases, as it combines therapeutic effects with a more enjoyable inhalation experience.

The inhaler may be a hand-held inhaler. This has the advantage of providing a portable, versatile, and cost-effective solution. This may offer convenience, flexibility, and ease of mounting.

It may be appreciated that the aroma fluid may not be released into the surrounding air in order to have an ortho-nasal effect on the user. Rather, it may be consumed substantially orally by the user when inhaling. Thereby, the aroma fluid may be perceived retro-nasally. This may have the advantage to provide a sensory impression of a taste perception. It is noted that as the olfactory sensory impression makes up a significant part of the gustatory perception when inhaling, the inhaler as described in here has the particular advantage to convert any substance to be inhaled into a joyful experience in an unprecedented manner.

According to a **2^{nd} embodiment,** the inhaler is configured such that the main fluid and the aroma fluid are configured to mix essentially within the mouthpiece.

The advantage of this embodiment may be multifaceted including but not limited to an improved mixing, which may lead to an improved taste. The two fluids, one carrying the medication and the other carrying the aroma, may come together and blend before being inhaled by the user. This mixing mechanism within the mouthpiece may ensure that the user receives a combined inhalation experience, where the therapeutic effects of the medication are complemented by the sensory experience of the aroma. This feature enhances the overall user experience by potentially making the inhalation process more pleasant. It is also not precluded that the inhalation may possibly be more effective, as the aroma could have additional therapeutic or calming effects.

According to a **3^{rd} embodiment,** the aroma channel is configured to provide a fluid communication between the aroma source and the mouthpiece.

The aroma channel may thus be structurally designed to allow the aroma component, which is generated from the aroma source, to travel through the aroma channel and reach the mouthpiece. This may ensure that when the user inhales through the mouthpiece, the aroma fluid is effectively delivered to the user's mouth. This may allow for a direct fluid communication pathway between the aroma source and the mouthpiece, which enhances the inhalation experience by ensuring that the aroma component is consistently and efficiently delivered to the user. This structural arrangement not only supports the primary function of delivering the medication substance but also adds an additional sensory dimension by incorporating the aroma component. This can be particularly beneficial in making the inhalation process more pleasant or therapeutic, especially for users with chronic respiratory diseases who may require frequent use of the inhaler.

### Structural main components

According to a **4^{th} embodiment,** the inhaler is further comprising a nebulizer membrane configured to convert a liquid substance, in particular a liquid medication substance, accommodated in the reservoir into an aerosol for being provided to the mouth of the user when the user sucks on the mouthpiece.

The introduction of a nebulizer membrane adds an enhancement to the inhaler's functionality. The nebulizer membrane is configured to convert a liquid substance, particularly a liquid medication, accommodated in the reservoir into an aerosol form. This transformation is helpful because it enables the liquid medication to be effectively inhaled by the user as an aerosol, which is a more suitable form for respiratory delivery.

The mechanism of communication between the components involves the nebulizer membrane interacting directly with the liquid substance in the reservoir. When the user sucks on the mouthpiece, the nebulizer membrane may be activated to convert the liquid into an aerosol, ensuring that the medication is efficiently delivered to the user's mouth for inhalation.

This enhances the inhaler's capability by allowing it to handle liquid medications and convert them into an aerosol form, which is helpful for effective respiratory treatment.

According to a **5^{th} embodiment,** the inhaler is further comprising measurement means for measuring performance data associated with the user.

This embodiment introduces a mechanism for monitoring and recording various parameters related to the user's interaction with the inhaler. The measurement means could include sensors, data processors, and possibly communication modules that work together to gather and analyze data such as the frequency and duration of inhalation, the volume of the substance inhaled, and the user's breathing patterns. These components may communicate through integrated circuits and/or wireless technology to ensure real-time data collection and transmission.

The inclusion of measurement means may enhance the inhaler's functionality by allowing for personalized monitoring and feedback, which can be helpful for managing chronic respiratory diseases. This data can be used to adjust the dosage of the medication substance, ensuring that the user receives the optimal amount for their condition.

Additionally, the performance data can be shared with healthcare providers, enabling them to track the patient's adherence to the prescribed treatment regimen and make informed decisions about their care.

According to a **6^{th} embodiment,** the measurement means comprise a spirometer for measuring lung volume during inhalation and/or exhalation of the user.

The spirometer may serve as a specific mechanism of communication between the user's respiratory activity and the inhaler's functionality. By incorporating a spirometer, the inhaler can provide real-time feedback on the user's lung volume, which can be helpful for monitoring the effectiveness of the inhalation process and ensuring that the user is receiving the correct dosage of the substance. This enhances the inhaler's utility by allowing for a more personalized and accurate delivery of the medication substance, particularly beneficial for users with chronic respiratory diseases who may require precise monitoring of their lung function.

Additionally, this feature can help in adjusting the inhalation technique or the dosage of the substance based on the measured lung volume, thereby optimizing the therapeutic outcomes. The integration of a spirometer into the inhaler represents an advancement in the design and functionality of inhalation devices, providing a more sophisticated tool for respiratory therapy.

According to a **7^{th} embodiment,** the inhaler, preferably the mouthpiece, comprises an exhaust valve for exhaled air, preferably bypassing the aroma channel.

This exhaust valve has the function to manage the exhalation process of the user, allowing the exhaled air to exit the inhaler efficiently. The exhaust valve may be arranged at any portion of the inhaler, e.g., it could be arranged at the distal or the proximal end, it may additionally or alternatively include one or more exhaust valve elements serving the purpose of enabling fluid to exit.

The exhaust valve may be strategically positioned to preferably bypass the aroma channel, ensuring that the exhaled air does not interfere with the aroma fluid pathway. This design consideration is beneficial as it maintains the integrity and purity of the aroma fluid being delivered to the user. By bypassing the aroma channel, the exhaust valve prevents any potential backflow or contamination of the aroma fluid with exhaled air, which could otherwise alter the user's experience or the effectiveness of the aroma component.

Primarily, the exhaust may enhance the ergonomic functionality of the device by providing a dedicated pathway for exhaled air, which can improve the user's comfort and ease of use. This is beneficial in a modular approach, where components can be easily assembled or disassembled, allowing for customization or maintenance.

The exhaust valve's placement in the mouthpiece also contributes to a more compact and integrated design, reducing the need for additional external components or pathways.

According to an **8^{th} embodiment,** the inhaler is further comprising an adapter comprising the mouthpiece.

The adapter may act as a mechanism of communication between the mouthpiece and the inhaler, such as, the rest of the inhaler components. The inclusion of the adapter brings several new features and advantages. Firstly, it provides a modular design that can facilitate easier assembly and disassembly of the inhaler, allowing for more convenient cleaning and maintenance. Secondly, the adapter can potentially enable compatibility with various types of mouthpieces, offering flexibility and customization for different user preferences or medical requirements. Thirdly, the adapter may enhance the structural integrity and stability of the connection between the mouthpiece and the inhaler body, ensuring a secure and airtight fit that is crucial for the effective delivery of both the main fluid and the aroma fluid.

Further, the adapter could also incorporate additional functionalities, such as filters or valves, to further improve the inhalation experience and the efficiency of substance delivery.

According to a **9^{th} embodiment,** the inhaler is further comprising a housing having an elongated shape, preferably an elongated cylindrical shape.

The housing may serve as the structural framework that encapsulates one or more components of the inhaler, including, e.g., the mouthpiece, aroma channel, aroma source, and reservoir.

The elongated shape, particularly the cylindrical form, provides ergonomic advantages, making the inhaler easier to handle and use. This design can enhance the user's grip and comfort during use, potentially improving the overall user experience. The elongated cylindrical shape may also contribute to the efficient internal arrangement of components, ensuring that the aroma channel, reservoir, and other parts are optimally positioned within the housing. This configuration can facilitate a smooth flow of both the main fluid and the aroma fluid, ensuring that the user receives a consistent and effective dose of the medication and aroma components.

According to a **10^{th} embodiment,** the elongated shape has a largest dimension and a smallest dimension, wherein the largest dimension is larger than the smallest dimension by a factor of at least 1.1, preferably at least 1.2, more preferably at least 1.5, even more preferably at least 2.0, most preferably by a factor of at least 2.5.

With this embodiment, a ratio is defined where the largest dimension exceeds the smallest dimension by a factor of at least 1.1, with a preference for this factor to be at least 1.2, more preferably at least 1.5, even more preferably at least 2.0, and most preferably by a factor of at least 2.5. This ensures the provision of a particular form factor that could influence the inhaler's usability, ergonomics, and possibly its performance. The elongated shape may facilitate easier handling and operation by the user, potentially improving the overall experience when using the inhaler.

The specific ratio between the largest and smallest dimensions is attributable to a deliberate design choice to ensure that the inhaler is not only functional but also comfortable to use, possibly enhancing the user's ability to effectively inhale the medication substance and aroma fluid. This design consideration could also impact the inhaler's portability, allowing it to be easily carried by the user without compromising its functionality. The elongated shape may also contribute to the internal arrangement of components within the inhaler, such as the mouthpiece, aroma channel, and reservoir, ensuring that they are optimally positioned to deliver the main fluid and aroma fluid efficiently.

This ratio of the largest dimension and a smallest dimension may also have advantages for the manufacturing process, potentially simplifying production by providing clear guidelines on the inhaler's dimensions.

According to an **11^{th} embodiment,** the reservoir is configured to accommodate a liquid and/or powdered medication.

The inhaler described includes a reservoir designed to accommodate either a liquid or powdered medication, enhancing its versatility and functionality. This configuration allows the inhaler to be used with a variety of medication forms, thereby broadening its application for different treatment needs. The reservoir's ability to hold both liquid and powdered forms of medication means that it can adapt to the specific requirements of various medications, whether they need to be dissolved or suspended in a liquid medium or administered in a dry, powdered state. This flexibility is particularly beneficial for treating a wide range of respiratory conditions, as it allows healthcare providers to prescribe the most effective form of medication for each individual patient.

According to a **12^{th} embodiment,** the inhaler is further comprising a mounting portion for releasably mounting the aroma source.

The specific mechanisms of communication between components include the mounting portion for releasably mounting the aroma source. This brings the advantage of allowing the aroma source to be easily attached and detached from the inhaler. This flexibility in mounting the aroma source enhances the usability and convenience of the inhaler, as it enables users to replace or change the aroma source as needed without difficulty. The releasable mounting mechanism ensures that the aroma source can be securely attached during use and easily removed when necessary, providing a practical solution for maintaining or customizing the aroma component of the inhaler. This feature also potentially allows for a variety of aroma sources to be used interchangeably, catering to different user preferences or therapeutic needs.

According to a **13^{th} embodiment,** the mounting portion comprises a mounting element configured to engage with a counterpart element of the aroma source, when the aroma source is mounted, the counterpart element preferably shaped complementary to the mounting element, the mounting element being preferably configured to substantially prevent rotation of the aroma source when mounted.

Any kind of mounting element and/or counterpart element known may be used in order to engage with the aroma source. For instance, the mounting element may comprise a protrusion or the like, wherein the counterpart element may comprise a corresponding recess or aperture so as to provide for a form-fitting mounting.

The aroma source may be mounted on the mounting portion in a movable manner (e.g., removable or releasable). This has the advantage to easily replace the aroma source in case a different taste is desired and/or in case the aroma source is substantially depleted. Nonetheless, it is not precluded that mounting of the aroma source on the adapter may be stationary (e.g., permanent or fixed).

According to a **14^{th} embodiment,** the inhaler is modular.

The mechanisms of communication between components in a modular inhaler may involve standardized connectors or interfaces that allow for the seamless integration of different component. For instance, the mouthpiece, aroma channel, and reservoir could each be separate modules that connect through means such as interlocking parts, snap-fit mechanisms, and/or threaded connections.

This modular design enhances the ease of maintenance and cleaning, as users can disassemble the inhaler to access and clean individual components thoroughly. It also allows for customization and flexibility, enabling users to replace or upgrade specific parts without needing to purchase an entirely new inhaler. Moreover, modularity can improve the overall durability and lifespan of the inhaler, as worn-out or damaged components can be replaced individually rather than discarding the entire device. Additionally, this feature may facilitate the production and distribution process, as manufacturers can produce and stock individual modules rather than complete inhalers, potentially reducing costs and improving inventory management.

### Smart features

According to a **15^{th} embodiment,** the inhaler is further comprising a control unit configured to track data, such as usage data and/or lung function data.

It is noted that the communication between the control unit and the other components of the inhaler may be facilitated through integrated sensors and data transmission pathways. These sensors could be strategically placed within the inhaler to detect and measure specific metrics, such as the frequency and duration of inhalation, the volume of the substance being inhaled, and the user's lung function parameters. The control unit may process this data, potentially using algorithms to analyze the information and provide feedback or alerts to the user. This may enable personalized monitoring and management of respiratory conditions. By tracking usage data, the control unit can help ensure that the user adheres to their prescribed treatment regimen, providing reminders or notifications if the inhaler is not being used as directed.

According to a **16^{th} embodiment,** the control unit is configured to alert a user if the amount of the substance accommodated in the reservoir is below a threshold.

The control unit may determine whether the level of the substance accommodated in the reservoir has fallen below the specified threshold. If the threshold is breached, the control unit may trigger an alert to the user. This alert can be communicated through various means, such as an auditory signal, a visual indicator, or a combination of both, depending on the design and intended use of the inhaler. This addresses practical concern for users who rely on the inhaler for regular medication, ensuring they are not caught unaware by an empty or near-empty reservoir. This may be important in the context of treating chronic respiratory diseases, where consistent and timely administration of medication is crucial for effective management of the condition. It is also viable that, as mentioned elsewhere herein, the reservoir corresponds to a channel or the like for merely guiding, not necessarily storing at least the substance. As understood, the control unit may be capable of alerting the user also when the substance in this channel or the like meets a certain threshold or is below a certain threshold.

According to a **17^{th} embodiment,** the inhaler is further comprising a communication module for transmitting measured data to a receiver, in particular to a smart device for being processed in a program, such as a companion application of the smart device.

The inclusion of this communication module introduces an enhancement to the inhaler's functionality by allowing for real-time data transmission and/or remote monitoring. This is beneficial for users and/or healthcare providers, as it facilitates the tracking of inhaler usage, adherence to prescribed medication schedules, and the monitoring of the effectiveness of the treatment.

According to an **18^{th} embodiment,** the inhaler is further comprising indication means for indicating the user to perform an action and/or to change or maintain an action.

These indication means serve the purpose of signaling the user to perform an action or to change or maintain an action. The new feature brought by the indication means enhances the usability and effectiveness of the inhaler by providing feedback or instructions to the user. This can be particularly beneficial in ensuring proper usage, optimizing the delivery of the medication, and improving the overall user experience.

According to an **19^{th} embodiment,** the communication module is configured to receive data from a smart device, wherein the indication means is configured to indicate the user to perform an action and/or to change or maintain an action based on the received data.

This feature introduces an interactive element to the inhaler, where the user is guided to perform specific actions and/or to alter or maintain their current behavior. The indication means may be implemented in various forms, such as visual indicators like lights or displays, auditory signals such as beeps or spoken instructions, or even haptic feedback like vibrations. The influence of the user behavior based on data, in particular external data, introduces a dynamic and adaptive aspect to the inhaler's operation.

According to an **20^{th} embodiment,** the received data comprises past performance data of the user, an average of past performance data of a period of time, past performance data of a plurality of users, an average of past performance data of a period of time of a plurality of users, and/or information of the user, such as age, weight, gender.

The past performance data generally allows for comparative analysis and benchmarking against a broader user base. This comparative data can be further refined by averaging the past performance data over a period of time for multiple users, offering insights into common trends and variations in the use of the inhaler across different demographics. Furthermore, the received data incorporates specific information about the user, such as age, weight, and gender, which are helpful parameters that can influence the effectiveness of the inhaled substance and the user's overall respiratory health.

### General features

According to a **21^{st} embodiment,** the inhaler is portable.

By being portable, the inhaler allows users to carry it easily and use it whenever necessary, which is particularly beneficial for individuals with chronic respiratory diseases who may need to use the inhaler multiple times a day. The hand-held nature of the inhaler ensures that it can be operated with ease and without requiring a stationary setup, thus providing flexibility and mobility to the user.

### Set and system

A **22^{nd} embodiment** of the invention is directed to an inhaler set for providing a substance to a user for inhaling, in particular a medication substance such as a substance for the treatment of respiratory diseases, in particular chronic respiratory diseases, the inhaler set comprising:
- an inhaler according to any one of the embodiments described in here; and
- an aroma source, in particular an aroma container, having an aroma chamber for accommodating an aroma component, the aroma source preferably mounted on a mounting portion of the inhaler.

The inhaler set for providing a substance to a user for inhaling may include all aspects and/or embodiments and/or examples and/or advantages described elsewhere herein, as it comprises the inhaler.

According to a **23^{rd} embodiment,** the aroma source is arranged at a proximal end of the inhaler or at a distal end of the inhaler.

This arrangement may provide flexibility in the placement of the aroma source, which can enhance the user experience by allowing customization based on user preference or specific design considerations.

By allowing the aroma source to be placed at either end of the inhaler, the design can accommodate different inhaler configurations and user needs. For instance, placing the aroma source at the proximal end might allow for a more immediate aroma experience as the user inhales, potentially enhancing the therapeutic or sensory effect of the inhalation process. Conversely, positioning the aroma source at the distal end might be beneficial for designs where the aroma is intended to be more subtle or where the proximal end is occupied by other components or features.

A **24^{th} embodiment** of the invention is directed to a system comprising:
- an inhaler according to any one of the embodiments 1 to 21 or an inhaler set according to embodiment 22 or 23, and
- a smart device, in particular a smartphone or a smart watch.

The system may include all aspects and/or embodiments and/or examples and/or advantages described elsewhere herein, as it comprises the inhaler or the inhaler set.

According to a **25^{th} embodiment,** the smart device comprises output means, such as a display, for outputting measured data and/or processed data to a user.

The system described includes a smart device, such as a smartphone or a smart watch, which comprises output means like a display. This display may be used for outputting measured data and/or processed data to a user. The smart device may process data and then utilizes its output means to present the information to the user. This may provide real-time feedback and detailed information to the user through a visual interface, enhancing the user experience and potentially improving the management of the inhaler's use. The integration of a display on the smart device means that users can easily access and interpret the data without needing additional equipment or technical knowledge.

According to a **26^{th} embodiment,** the aroma channel may be comprised at least partially, preferably in its entirety, by the mouthpiece. Preferably, the aroma channel may run essentially along the mouthpiece, particularly within the mouthpiece. The aroma channel may end essentially at a proximal end of the mouthpiece.

The term "substantially" as used in the present disclosure means that manufacturing tolerances are included. Thus, slight variations of dimensions, shapes, or the like deviating from their intended dimensions, shapes, or the like are encompassed. Thereby, the present disclosure is not limited to the precise dimensions, shapes, or the like mentioned in here.

### Brief description of the figures

In the following, preferred embodiments are described, by way of example only. Reference is made to the following accompanying figures:
- Fig. 1: illustrates an inhaler according to a general embodiment of the invention;
- Fig. 2: illustrates an inhaler according to an embodiment of the invention in cross-sectional perspective view;
- Fig. 3: illustrates the inhaler of Fig. 2 mounted on a holding element;
- Fig. 4: illustrates an aroma source and a mouthpiece of the inhaler of Figs. 2 and 3;
- Fig. 5: illustrates a system of an inhaler and a smart device according to an embodiment of the invention.
- Fig. 6: illustrates an inhaler according to an embodiment of the invention.

### Detailed description of preferred embodiments

### Definitions

The term "aroma component" or equivalents as used in the present disclosure may include materials that may be carried with the help of another fluid, such as air. For instance, the aroma component may be carried as particles. Additionally, or alternatively, the aroma component may volatilize. Thereby, a volatilized component in the form of an aerosol may be provided. The volatilized component may mix with another fluid, such as air.

The aroma component may include any material, in particular materials that contribute to scents like chocolate orange, bubble gum, blue raspberry, kola, watermelon, orangeade, apple, pineapple. Further, the aroma component may include materials that contribute to scents of fruit extracts, vanilla, coffee aroma compounds, tea essence, herbal infusions, spices, hops, malt extracts, wine aroma compounds, and carbonation aroma.

The aroma component may be provided in any desired form including but not limited to a solid, a liquid, a gel or a wax or any other suitable form. Said aroma component may also be a combination or a blend of materials.

The terms "one end", "the other end", "outer side", "upper", "above", "inner side", "under", "below", "horizontal", "coaxial", "central", "end" "part", "length", "outer end" etc., which indicate the orientation or positional relationship, are based on the orientation or positional relationship shown in the drawings.

The terms "upper", "above", "below", "under" and the like as used in the present invention to indicate a relative position in space are used for the purpose of facilitating explanation to describe an inhaler, adapter, device, part, component and/or feature shown in the drawings relative to the relationship of another inhaler, adapter, device, part, component and/or feature.

The term of the relative position in space may be intended to include different orientations of the inhaler, adapter, device, part, component and/or feature other than those shown in the figures. For example, if the inhaler, adapter, device, part, component and/or feature in the figure is turned over, the inhaler, adapter, device, part, component and/or feature described as being "below" or "under" other inhalers, adapters, devices, parts, components and/or features will be "above" the other inhaler, adapter, device, part, component and/or feature. Therefore, the exemplary term "below" can encompass both the above and below orientations.

### Embodiments shown in the figures

In the following, the invention is described with reference to the accompanying figures in more detail. However, the present invention can also be used in other embodiments not explicitly disclosed hereafter. As detailed below, the embodiments are compatible with each other, and individual features of one embodiment may also be applied to another embodiment.

Throughout the figures and description, the same reference numerals refer to the same elements, unless stated otherwise. The figures may not be drawn to scale, and the relative size, proportions, and depiction of elements in the figures may be exaggerated for the purpose of clarity, illustration, and convenience. The figures do not limit the scope of the claims but merely support the understanding of the invention.

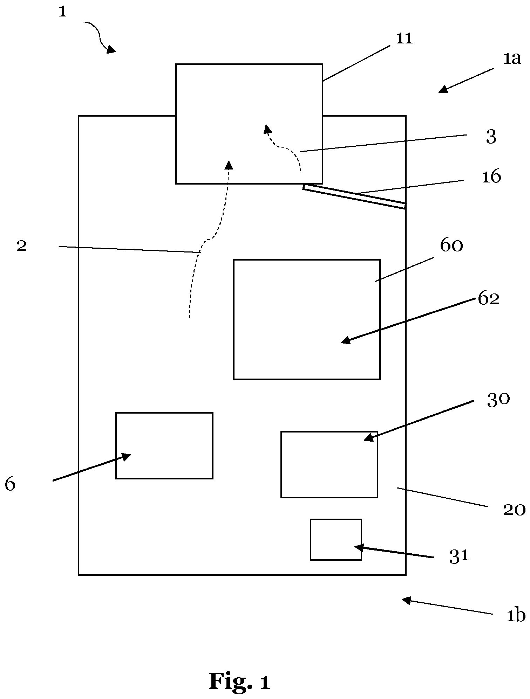

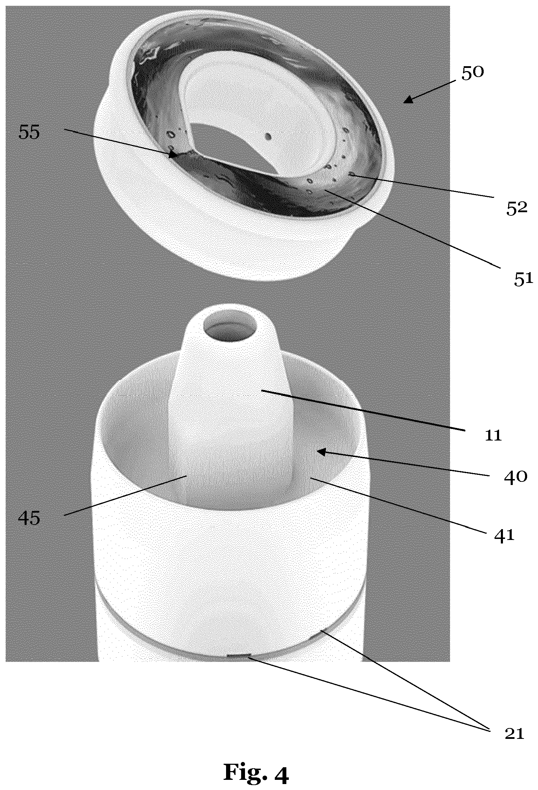

Fig. **1** illustrates a general embodiment of an inhaler 1 for providing a substance 62, such as a medication substance 62, to a user for inhalation. The inhaler 1 comprises a mouthpiece 11. It may be located at the proximal end 1a of the inhaler 1 but could be arranged at any other portion of the inhaler 1. The user inhales through the mouthpiece 11, which may be in communication with the aroma channel 16 and the main fluid 2. The aroma channel 16 configured for being connected with an aroma source 50 (not shown in **Fig. 1****,** but in **Fig. 4** in greater detail), allowing an aroma fluid 3 to be delivered to the user. The aroma fluid 3 is based on a gas, such as air, and comprises an aroma component 52 generated from the aroma source 50.

The main fluid 2 may travel along a main fluid path (exemplarily also indicated by reference numeral 2) and is a mixture of air and the substance 62. The substance 62 can be accommodated in a reservoir 60, which may be arranged within the housing 20 of the inhaler 1. The reservoir 60 can accommodate, e.g., a liquid and/or powdered medication. The substance 62 may be pre-mixed, e.g., with air and arranged within the reservoir 60. The reservoir 60 may take the form of a channel or the like.

The inhaler 1 also features measurement means 6, which can take various forms, and which is used to measure performance data associated with the user. This can include a spirometer for measuring lung volume during inhalation and/or exhalation. The measurement data can be tracked and processed by, e.g., a control unit 30, which may be housed within the inhaler 1. The control unit 30 is configured to track various data, such as usage data and lung function data, and can alert the user, e.g., if the amount of the substance 62 in the reservoir 60 falls below a certain threshold.

The measurement means 6 may provide an opportunity for users to engage more actively in their treatment, as they can receive essentially immediate feedback on their inhalation technique and make necessary adjustments. This can lead to improved treatment outcomes and greater user satisfaction. Furthermore, the data collected can be used for research purposes, contributing to a better understanding of respiratory diseases and the effectiveness of various treatments. Thereby, the integration of measurement means 6 into the inhaler 1 represents an advancement in the management of respiratory diseases, offering benefits for both users and healthcare providers through enhanced monitoring, personalized treatment, and valuable data collection. The measurement means 6 may continuously measure performance data associated with the user.

As to the performance data, various types of performance data may be encompassed, including but not limited to delivery efficiency, which could represent the proportion of medication delivered to the lower respiratory tract, and particle size distribution, potentially characterized by the mass median aerodynamic diameter (MMAD), as this may influence pulmonary deposition. Flow rate sensitivity and actuation force might also be measured to assess the inhalation effort and physical activation required for use. Further, dose consistency could provide an indication of the variability in the medication dispensed, while residual medication data may reflect the quantity of unused substance remaining in the device. Lung volume may also be a performance data. Additional performance data might include leakage rates, environmental tolerance under conditions such as varying temperature and humidity, and usability parameters derived from user feedback, which could provide insights into the inhaler's operability and user satisfaction. These performance data could collectively serve as a basis for assessing and improving inhaler functionality and design.

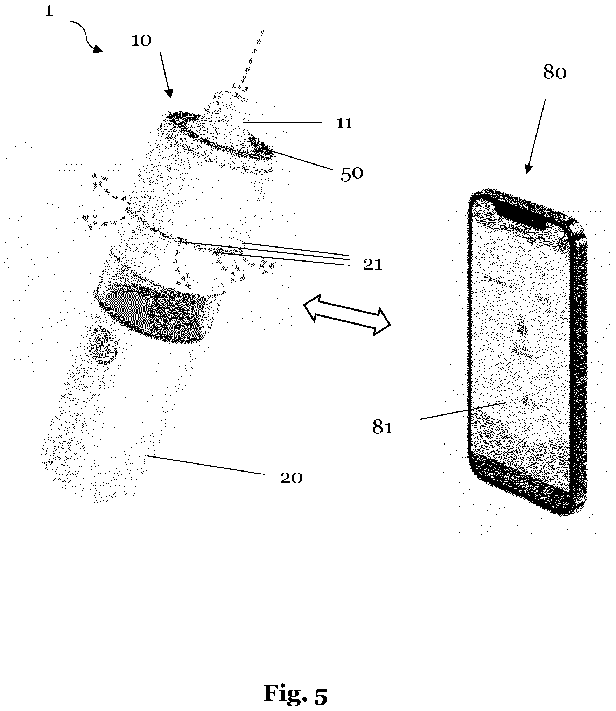

Additionally, the inhaler 1 may include a communication module 31, which allows for the transmission of measured data to a receiver, such as a smart device 80 (not shown in **Fig. 1****,** but depicted in **Fig. 5** in greater detail). This enables the data to be processed in a companion application on the smart device 80. This may provide the user with detailed feedback and insights into their inhalation patterns and medication usage.

The inhaler 1 presented in **Fig. 1** generally shows various functionalities, components and their various interconnections, which can be implemented in various ways. The inhaler 1 is designed to deliver both a medication substance 62 and an aroma fluid 3 to the user, with integrated measurement and communication capabilities to enhance user experience and ensure effective medication delivery.

The particular benefits provided by the inhaler 1 are that it can incorporate retronasal taste technology to make the inhalation process more pleasant by allowing patients to experience different flavors, thereby increasing their motivation to adhere to their therapy regimen. As described elsewhere in greater detail, the inhaler 1 may be equipped with smart features that can track usage and lung function data, transmitting this data to a companion application for reminders, progress tracking, and data sharing with healthcare providers. Its general arrangement may aid in reducing the stigma associated with using medical devices in public. Its general arrangement may be user-friendly, making it easy to handle and use.

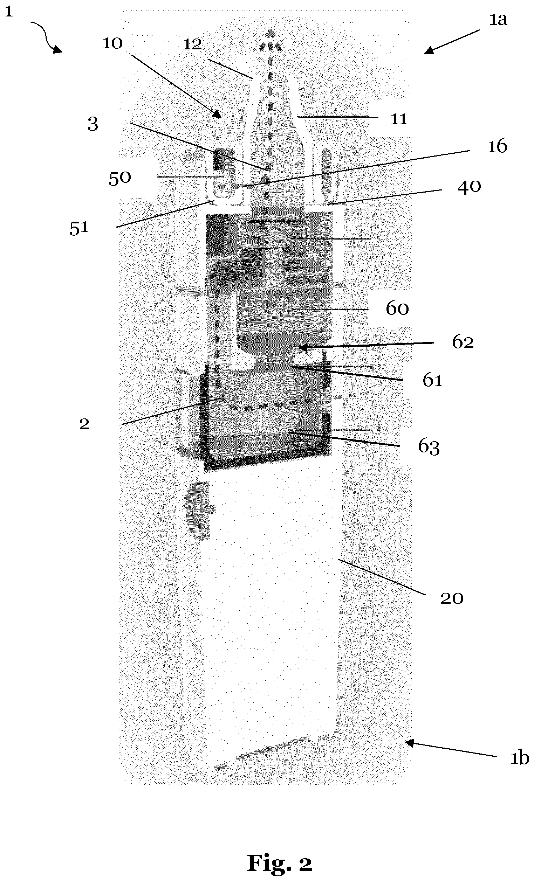

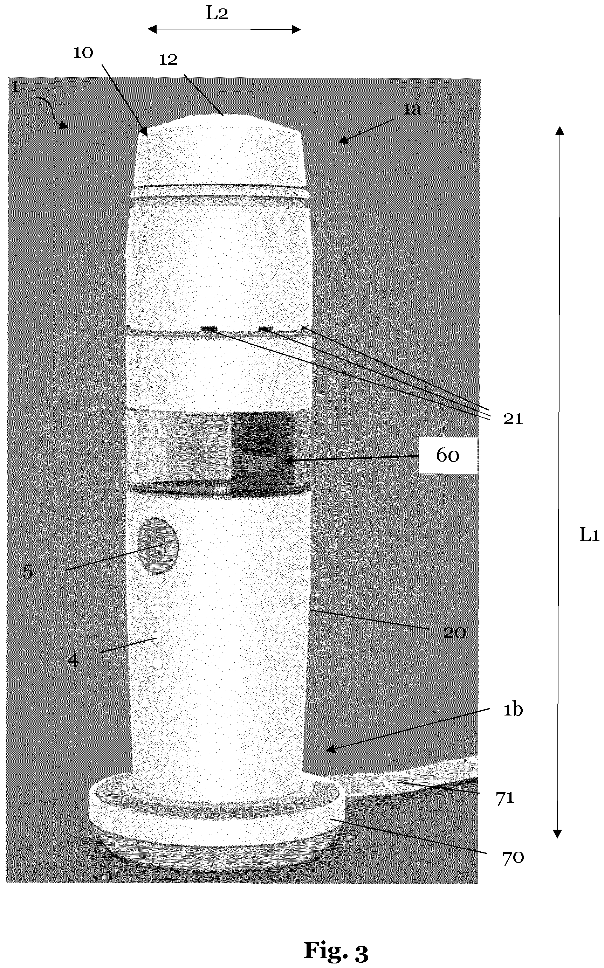

**Figs. 2** **and** **3** illustrate an embodiment of an inhaler 1 designed to provide a substance 62, such as a medication, to a user for inhalation. For brevity only, not all of the reference numerals described elsewhere herein (e.g., reference numerals of **Fig. 1**) are repeated in the following description for Figs. 2 and 3. As can be seen, the inhaler 1 comprises a mouthpiece 11, an aroma channel 16, a reservoir 60, and a housing 20. The housing 20 of the inhaler 1 is depicted as having an elongated shape. Preferably, it may be cylindrical, but it is not limited to such a shape. This design is ergonomic, making the inhaler 1 portable and easy to handle.

Specifically, such a design of the housing 20 may contribute to the durability and protection of the internal components, safeguarding them from damage and ensuring the inhaler's longevity. Overall, the inclusion of a housing 20 with an elongated, preferably cylindrical shape, brings ergonomic, functional, and aesthetic enhancements to the inhaler 1, improving its usability, efficiency, and user appeal. Additionally, the elongated shape can provide aesthetic benefits, giving the inhaler a sleek and modern appearance that may be more appealing to users.

The housing 20 has a proximal end 1a and a distal end 1b, with the proximal end 1a being essentially at the top part of the inhaler 1 in **Figs. 2** **and** **3** where the mouthpiece 10 is located. The mouthpiece 10 may have a mouth end 12. The mouthpiece 10 is integral to the inhaler's function, allowing the user to inhale the substance. The inhaler may also have a power button 5, which can be implemented in various ways.

As can be seen, the mouthpiece 11 is positioned at the proximal end 1a of the inhaler 1, where the user places their mouth to inhale the substance. The aroma channel 16 is connected to an aroma source 50, which is mounted on a mounting portion 40. The aroma source 50 contains an aroma component 52, which is released into the aroma channel 16 when the user inhales. The aroma channel 12 may be arranged adjacent to the mouthpiece 10, which facilitates the connection with an aroma source 50. The aroma channel 12 is designed to deliver an aroma fluid to the user, enhancing the inhalation experience.

The reservoir 60 is designed to accommodate the substance 62. The reservoir 60 is equipped with a nebulizer membrane 61, which converts the liquid substance into an aerosol, facilitating inhalation by the user. The nebulizer membrane 61 is activated when the user sucks on the mouthpiece 11, ensuring that the aerosolized medication is delivered efficiently.

The nebulizer membrane 61 may be a vibrating membrane that operates at preferred frequencies. Exemplary frequencies range between 1 MHz and 3 MHz. This may allow producing fine aerosol droplets.

The aerosol may then form the main fluid 2, based on a gas such as air and the substance. It is likewise possible that the main fluid 2 based on a gas such as air is already mixed with the substance 62 outside the inhaler 1. Thereby, pre-mixed main fluids 2 are encompassed for being provided to the mouth of the user for inhaling the substance 62 through the mouthpiece 11. It is viable that the inhaler 1 features a reservoir 60 visible through an essentially transparent or semi-transparent section of the housing 20 (see **Fig. 3**).

The housing 20 of the inhaler 1 may have an elongated cylindrical shape, with the largest dimension L1 (see **Fig. 3**) being larger than the smallest dimension L1 (see **Fig. 3**). This design facilitates easy handling and portability, making the inhaler 1 a hand-held device. The largest dimension L1 can be larger than the smallest dimension L2 by a factor of at least 1.1, preferably at least 1.2, more preferably at least 1.5, even more preferably at least 2.0, most preferably by a factor of at least 2.5.

Also shown is an adapter 10 in greater detail, which may include the mouthpiece 11. However, the mouthpiece may alternatively be provided separately to the inhaler 1. The adapter 10 can ensure a secure connection between the mouthpiece 11 and the rest of the inhaler 1. The inhaler 1 also features an exhaust valve 21, which allows exhaled air to bypass the aroma channel 16. This can ensure that the user only inhales the intended mixture of the main fluid 2 and the aroma fluid 3.

By integrating the mouthpiece 11 into an adapter 10, the design not only simplifies the overall construction but also enhances the functionality and user experience of the inhaler, making it a more versatile and user-friendly device for administering respiratory treatments.

The aroma fluid 3, which is based on a gas such as air and contains the aroma component 52, is generated from the aroma source 50. For this, the aroma channel 16 is configured to provide a fluid communication between the aroma source 50 and the mouthpiece 11. The aroma fluid 3 is directed through the aroma channel 16 and mixed with the main fluid 2 within the mouthpiece 11. This mixture is then inhaled by the user, providing both the medication and the desired aroma. The inhaler 1 can be configured such that the main fluid 2 and the aroma fluid 3 are configured to mix essentially within the mouthpiece 11.

As can be gathered inter alia from the cross-section in **Fig. 2**, the inhaler 1 is modular, allowing for easy replacement or upgrading of individual components. This modular design enhances the versatility and usability of the inhaler 1, making it suitable for a wide range of applications and user needs. The modular inhaler 1 may additionally facilitate a hygienic design that allows for easy disassembly and cleaning of the modular components of the inhaler 1.

The modularity of the inhaler 1 also opens up possibilities for future enhancements and innovations. For instance, new components of the inhaler 1 with advanced features can be developed and integrated into the existing inhaler 1. For instance, a module with a digital interface for monitoring usage or a module with enhanced filtration capabilities could be introduced. In addition, existing components of the inhaler 1 could be easily replaced.

As can be further seen, the inhaler 1 may be received in a holding element 70, which may conform to the shape of the inhaler 1 at least partially. The holding element 70 may facilitate a connection between the inhaler 1 and a power source for charging the inhaler 1. This connection may be implemented by a connecting element 71, such as an electric cable.

**Fig. 4** illustrates an embodiment of an inhaler with a focus on the mounting portion 40 and the aroma source 50. For brevity only, not all of the reference numerals described elsewhere herein are repeated in the following description for Fig. 4. The mounting portion 40 may serve the purpose of releasably mounting the aroma source 50. The mounting portion 40 may have a mounting rim 41. The mouthpiece 11 may form part of the mounting portion 40. The mounting portion 40 may have any suitable shape for allowing mounting of the aroma source 50. The mounting portion 40 may provide structural stability and mechanical robustness to the aroma source 50. This may provide for a substantially firm attachment when mounted.

The mounting portion 40 comprises a mounting element 45 that engages with a counterpart element 55 of the aroma source 50, ensuring a secure and stable connection. The mounting element 45 is designed to substantially prevent rotation of the aroma source 50 when it is mounted, ensuring stability and proper alignment.

The counterpart element 55 preferably shaped to complement the mounting element 45 can ensure a secure and precise fit. This complementary shaping facilitates a reliable connection between the aroma source 50 and the mounting portion 40, enhancing the stability of the aroma source 50 when attached. The mounting element 45 is further configured to substantially prevent rotation of the aroma source 50 once it is mounted. This anti-rotation feature may ensure that the aroma source 50 remains in a fixed position during use, which is beneficial for maintaining consistent delivery of the aroma fluid to the user. By preventing rotation, the design minimizes the risk of misalignment or disconnection that could disrupt the flow of the aroma fluid through the aroma channel.

The specific configuration of the mounting element and its interaction with the counterpart element of the aroma source represent an advancement over prior designs that may not have addressed the potential issues of rotation or misalignment. This development is beneficial in the context of inhalers where precise delivery of both the main fluid containing the medication substance, and the aroma fluid may facilitate effective treatment and user satisfaction.

The aroma source 50 is depicted as a detachable component that can be mounted onto the inhaler 1. The aroma source 50 may be an aroma container 50. The aroma source 50 may comprise an aroma chamber 51 for accommodating the aroma component 52.

The aroma source 50 is shown separate from the inhaler 1, specifically from the mouthpiece 11, highlighting its detachable nature and the ease with which it can be mounted onto the inhaler 1. The aroma component 52 housed in the aroma chamber 51 of the aroma source 51 is intended to be mixed with the main fluid 2 when the user inhales through the mouthpiece 11, providing an enhanced sensory experience. The aroma channel is not shown in **Fig. 4****.**

As understood, an inhaler set for providing a substance 62 to a user for inhaling, in particular a medication substance 62 such as a substance 62 for the treatment of respiratory diseases, in particular chronic respiratory diseases, comprises an inhaler 1 as shown in any one of the embodiments in here, and an aroma source 50, in particular an aroma container 50, having an aroma chamber 51 for accommodating an aroma component 52, preferably mounted on a mounting portion 40 of the inhaler 1. As shown inter alia in **Fig. 4****,** the aroma source 50 can be arranged at a proximal end 1a (see **Fig. 3**) of the inhaler 1 or at a distal end 1b of the inhaler 1.

**Fig. 5** illustrates an embodiment of an inhaler system, comprising an inhaler 1 and a smart device 80. For brevity only, not all of the reference numerals described elsewhere herein are repeated in the following description for Fig. 5.

The aroma source 50 may be at least partially visible from outside as can be gathered from Fig. 5, which could indicate a user that the inhaler 1 has more functionality compared to inhalers of the prior art.

The smart device 80, which can be a smartphone, is shown in communication with the inhaler 1. This communication is indicated by way of an arrow pointing in two directions, showing the capability to transmit data in at least two ways. This communication is facilitated by a communication module 31 (see **Fig. 1**) within the inhaler 1, enabling data exchange between the inhaler 1 and the smart device 80. The smart device 81 may thus act as a receiver and/or the smart device 80 may comprise a receiver. The smart device 80 features output means, such as a display 81, which is used to present, among others, measured data and/or processed information to the user. This data can include usage statistics, lung function data, and alerts regarding the medication levels in the reservoir of the inhaler 1. Data, such as measured data may be processed in a program, such as a companion application of the smart device 80. The companion application may provide reminders for medication usage, may track therapy progress, and/or may allow data sharing with healthcare providers. The companion application may include a multi-factor authentication system to ensure the security of the patient's data.

The inhaler may further comprise indication means 4 (see **Fig. 3**) for indicating the user to perform an action and/or to change or maintain an action. Specifically, the indication means 4 may be configured to indicate the user to perform an action and/or to change or maintain an action based on the received data, e.g., data received by the communication module 31, wherein the data may be sent from the smart device 80. The indication means 4 may be implemented by LEDs, however, different arrangements are possible including but not limited to audio and/or haptic feedback.

As mentioned elsewhere herein, the inhaler 1 is capable of tracking and transmitting performance data to the smart device 80. E.g., data may then be processed by the smart device 80 and displayed and/or sent back to the inhaler 1. Further, this functionality may additionally or alternatively be managed by a control unit 30 of the inhaler 1, which may process the data collected by various measurement means 6 integrated in the inhaler 1. The control unit 30 can alert the user, e.g., if the substance 62, in particular the medication substance 62 level falls below a certain threshold, ensuring timely refills and continuous treatment.

Generally, received data, e.g., data that the inhaler 1 receives may comprise past performance data of the user, an average of past performance data of a period of time, past performance data of a plurality of users, an average of past performance data of a period of time of a plurality of users, and/or information of the user, such as age, weight, gender.

The essentially seamless communication and/or data presentation between the inhaler 1 and the smart device 80 adds a layer of convenience and functionality, making the system more user-friendly and efficient. The output means 81, such as a display, can also support various forms of data visualization, including graphs, alerts, and reminders, which can further aid users in tracking their inhaler usage patterns and maintaining their treatment regimen. Overall, this enhances the usability and effectiveness of the inhaler system by leveraging the capabilities of smart devices to provide accessible and actionable information to users.

The inhaler 1 described herein can be embodied in various forms and shapes to accommodate different user preferences and ergonomic requirements. The embodiments of the **Figs. 2** to **5** primarily describe an embodiment in which the inhaler may have an elongated cylindrical shape, which provides a comfortable grip and ease of use. The cylindrical form can include a tapered end, facilitating a more natural fit in the user's hand. In an embodiment, the inhaler 1 may take on a more compact, rectangular shape with rounded edges, enhancing portability and discreetness. This form factor can be particularly advantageous for users who require an inhaler 1 that can be easily carried in a pocket or small bag. Additionally, the inhaler 1 can be designed with a modular structure, allowing for interchangeable components such as mouthpieces, aroma reservoirs, and medication chambers. For instance, the mouthpiece can be detachable and available in various sizes and shapes to suit different user preferences, including a flat, wide mouthpiece for a more comfortable fit or a narrow, elongated mouthpiece for targeted delivery. The aroma source can be positioned at any location, e.g., at the proximal end or the distal end of the inhaler 1, depending on the desired airflow and user experience. Furthermore, the inhaler 1 housing 20 can be constructed with an ergonomic curvature to conform to the contours of the user's hand, providing a secure and comfortable grip. In some embodiments, the inhaler 1 may feature a foldable or collapsible design, allowing it to be compacted for storage and expanded for use. The inhaler 1 can also be equipped with a variety of surface textures, such as a smooth, glossy finish for a sleek appearance or a textured, matte finish for enhanced grip and tactile feedback. These various forms and shapes ensure that the inhaler 1 can be tailored to meet the diverse needs and preferences of users, thereby improving the overall user experience and adherence to therapy.

It is to note that any features relating to data transmission, any smart features or the like described herein (e.g., the one's associated with the smart device 80, the control unit 30 and/or the communication module 31) may be implemented in hardware, software, firmware, and/or combinations thereof. If implemented in software/firmware, the functions may be stored on or transmitted as one or more instructions or code on a computer-readable medium. Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor. For instance, the control unit 30 as described herein may be implemented in hardware, software, firmware, and/or combinations thereof, for example, by means of one or more general-purpose or special-purpose processors.

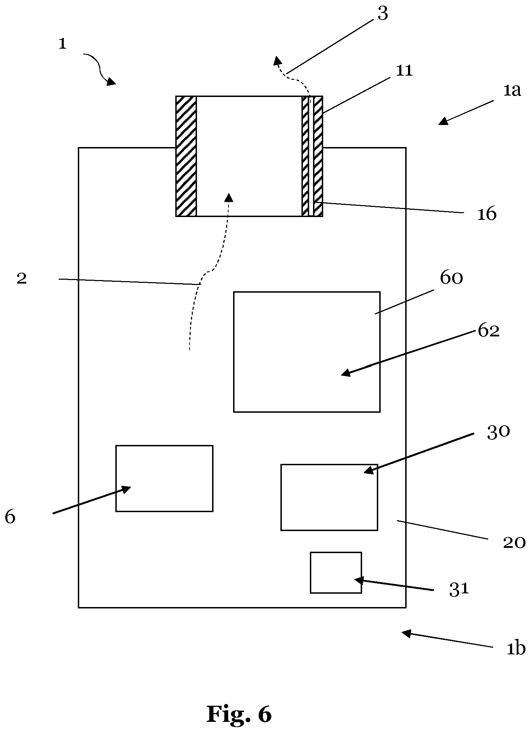

**Fig. 6** illustrates an embodiment of an inhaler 1 for providing a substance 62, such as a medication substance 62, to a user for inhalation. The inhaler 1 may comprise any features of the embodiments described elsewhere herein, particularly of the embodiments described with respect to **Figs. 1** to **5**. Compared to the embodiment of Fig. 1, the inhaler 1 comprises an aroma channel 16 extending essentially within the mouthpiece 11. While the aroma channel 16 is depicted to run along the entire mouthpiece 11, it can run along any distance thereof. Particularly, the aroma channel 16 may end at any position within the mouthpiece 11, and, e.g., then connect to the aroma source 50 (see e.g., **Fig. 2**), which may be situated in proximity to this end or further away. The aroma channel 16 may comprise one or more aroma channel segments that are connected to one another. Particularly, as shown in **Fig. 6****,** the aroma channel 16 may be comprised at least partially, preferably in its entirety, by the mouthpiece 11. Preferably, the aroma channel 16 may run essentially along the mouthpiece 11, particularly within the mouthpiece 11. The aroma channel 16 may end essentially at a proximal end of the mouthpiece 11.

It will be apparent to those skilled in the art that numerous modifications and variations of the described examples and embodiments are possible in light of the above teaching. The disclosed examples and embodiments are presented for purposes of illustration only. Other embodiments may include some or all of the features disclosed herein. Therefore, it is the intent to cover all such modifications and alternate embodiments as may come within the true scope of this invention.

### List of reference signs

- 1: inhaler
- 1a: proximal end
- 1b: distal end
- 2: main fluid
- 3: aroma fluid
- 4: indication means
- 5: power button
- 6: measurement means
- 10: adapter
- 11: mouthpiece
- 12: mouth end
- 16: aroma channel
- 20: housing
- 21: exhaust valve
- 30: control unit
- 31: communication module
- 40: mounting portion
- 41: rim
- 45: mounting element
- 50: aroma source
- 51: aroma chamber
- 52: aroma component
- 55: counterpart element of the aroma source
- 60: reservoir
- 61: nebulizer membrane
- 62: substance
- 63: nebulizer chamber
- 70: holding element
- 71: connecting element
- 80: smart device
- 81: output means
- L1: largest dimension
- L2: smallest dimension

## Claims

1. An inhaler (1) for providing a substance (62) to a user for inhaling, in particular a medication substance (62) such as a substance (62) for the treatment of respiratory diseases, in particular chronic respiratory diseases, the inhaler (1) comprising:
a mouthpiece (11),
an aroma channel (16) for being connected with an aroma source (50); and
a reservoir (60) for accommodating at least the substance (62);
wherein the inhaler (1) is configured such that, when a user sucks on the mouthpiece (11):
a main fluid (2) based on a gas such as air mixed with the substance (62) can be provided to the mouth of the user for inhaling the substance (62) through the mouthpiece (11); and
an aroma fluid (3) based on a gas such as air and comprising an aroma component (52) generated from the aroma source (50), can be provided to the mouth of the user via the aroma channel (16).

2. The inhaler (1) of the preceding claim, wherein the inhaler (1) is configured such that the main fluid (2) and the aroma fluid (3) are configured to mix essentially within the mouthpiece (11).

3. The inhaler (1) of any one of the preceding claims, wherein the aroma channel (16) is configured to provide a fluid communication between the aroma source (50) and the mouthpiece (11).

4. The inhaler (1) of any one of the preceding claims, further comprising a nebulizer membrane (61) configured to convert a liquid substance, in particular a liquid medication substance, accommodated in the reservoir (60) into an aerosol for being provided to the mouth of the user when the user sucks on the mouthpiece (11).

5. The inhaler (1) of any one of the preceding claims, further comprising measurement means (6) for measuring performance data associated with the user.

6. The inhaler (1) of any one of the preceding claims, wherein the inhaler (1), preferably the mouthpiece (11), comprises an exhaust valve for exhaled air, preferably bypassing the aroma channel (16).

7. The inhaler (1) of any one of the preceding claims, further comprising an adapter (10) comprising the mouthpiece (11).

8. The inhaler (1) of any one of the preceding claims, further comprising a housing (20) having an elongated shape, preferably an elongated cylindrical shape.

9. The inhaler (1) of the preceding claim, wherein the elongated shape has a largest dimension (L1) and a smallest dimension (L2),
wherein the largest dimension is larger than the smallest dimension by a factor of at least 1.1, preferably at least 1.2, more preferably at least 1.5, even more preferably at least 2.0, most preferably by a factor of at least 2.5.

10. The inhaler (1) of any one of the preceding claims, wherein the reservoir (60) is configured to accommodate a liquid and/or powdered medication.

11. The inhaler (1) of any one of the preceding claims, further comprising a mounting portion (40) for releasably mounting the aroma source (50).

12. The inhaler (1) of the preceding claim, wherein the mounting portion (40) comprises a mounting element (45) configured to engage with a counterpart element (55) of the aroma source (50), when the aroma source (50) is mounted, the counterpart element (55) preferably shaped complementary to the mounting element (45),
the mounting element (45) being preferably configured to substantially prevent rotation of the aroma source (50) when mounted.

13. The inhaler (1) of any one of the preceding claims, further comprising a control unit (30) configured to track data, such as usage data and/or lung function data.

14. An inhaler set for providing a substance (62) to a user for inhaling, in particular a medication substance (62) such as a substance (62) for the treatment of respiratory diseases, in particular chronic respiratory diseases, the inhaler set comprising:
- an inhaler (1) according to any one of the preceding claims; and
- an aroma source (50), in particular an aroma container (50), having an aroma chamber (51) for accommodating an aroma component (52), preferably mounted on a mounting portion (40) of the inhaler (1).

15. A system comprising:
- an inhaler (1) according to any one of the claims 1 to 13 or an inhaler set according to claim 14, and
- a smart device (80), in particular a smartphone or a smart watch.
